(19) Europäisches Patentamt
European Patent Office
Office européen des brevets

(11) **EP 3 825 414 B1**

(12) **EUROPEAN PATENT SPECIFICATION**

(45) Date of publication and mention
of the grant of the patent:
**28.08.2024 Bulletin 2024/35**

(51) International Patent Classification (IPC):
**G16B 20/20** (2019.01)    **C12Q 1/6869** (2018.01)
**C12Q 1/6883** (2018.01)

(21) Application number: **19758787.6**

(52) Cooperative Patent Classification (CPC):
**G16B 20/20;** C12Q 1/6869; C12Q 1/6883;
C12Q 2600/156

(22) Date of filing: **19.07.2019**

(86) International application number:
**PCT/ES2019/070506**

(87) International publication number:
**WO 2020/016477 (23.01.2020 Gazette 2020/04)**

(54) **METHOD FOR THE STUDY OF EMBRYO MUTATIONS IN VITRO REPRODUCTION PROCESSES**

VERFAHREN ZUM STUDIEREN VON EMBRYOMUTATIONEN IN
IN-VITRO-REPRODUKTIONSPROZESSEN

MÉTHODE POUR L'ÉTUDE DE MUTATIONS CHEZ DES EMBRYONS DANS DES PROCÉDÉS DE
REPRODUCTION IN VITRO

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.07.2018 ES 201830731**

(43) Date of publication of application:
**26.05.2021 Bulletin 2021/21**

(73) Proprietor: **Journey Genomics, S.L.
03202 Elche Alicante (ES)**

(72) Inventors:
• **ALCARAZ MAS, Luis A.
03202 Elche (Alicante) (ES)**
• **ANTÓN SALAS, Andrés
03202 Elche (Alicante) (ES)**

(74) Representative: **Pons
Glorieta Rubén Darío 4
28010 Madrid (ES)**

(56) References cited:
• **LIYING YAN ET AL: "Live births after
simultaneous avoidance of monogenic diseases
and chromosome abnormality by
next-generation sequencing with linkage
analyses", PROCEEDINGS OF THE NATIONAL
ACADEMY OF SCIENCES (PNAS), vol. 112, no.
52, 28 December 2015 (2015-12-28), US, pages
15964 - 15969, XP055625129, ISSN: 0027-8424,
DOI: 10.1073/pnas.1523297113**
• **GONZÁLEZ-REIG SANTIAGO ET AL: "New
all-in-one protocol for 24-chromosome
aneuploidies and monogenic diseases detection
by next- generation sequencing: first-year
experience", REPRODUCTIVE BIOMEDICINE
ONLINE, ELSEVIER, AMSTERDAM, NL, vol. 36, 14
February 2018 (2018-02-14), XP085349865, ISSN:
1472-6483, DOI: 10.1016/J.RBMO.2017.10.083**
• **NATHAN R. TREFF ET AL: "Advances in
Preimplantation Genetic Testing for Monogenic
Disease and Aneuploidy", ANNUAL REVIEW OF
GENOMICS AND HUMAN GENETICS, vol. 18, no.
1, 31 August 2017 (2017-08-31), US, pages 189 -
200, XP055639149, ISSN: 1527-8204, DOI:
10.1146/annurev-genom-091416-035508**

- **SUEOKA KOU: "Preimplantation genetic diagnosis: an update on current technologies and ethical considerations", REPRODUCTIVE MEDICINE AND BIOLOGY, SPRINGER JAPAN, TOKYO, vol. 15, no. 2, 14 November 2015 (2015-11-14), pages 69 - 75, XP035960666, ISSN: 1445-5781, [retrieved on 20151114], DOI: 10.1007/S12522-015-0224-6**

**Description**

The object of the present invention is a method for the study of embryo mutations in *in vitro* reproduction processes with the particular feature that it combines the detection techniques of Aneuploidy (PGD-A) and the study of monogenic diseases in embryos (PGD-M) according to claim 1.

**TECHNICAL FIELD**

The present invention relates to a method for the study of mutations in embryos of couples undergoing *in vitro* reproductive cycles by means of SNP (single nucleotide polymorphism) analysis by massive sequencing that combines the detection of Aneuploidies (PGD-A Preimplantation Genetic Diagnosis of Aneuploidies) and the study of monogenic diseases in embryos (PGD-M, Preimplantation Genetic Diagnosis for Monogenic Diseases) with a single biopsy.

**PRIOR ART**

A single nucleotide polymorphism or SNP is a variation in the DNA sequence that affects a single base (adenine (A), thymine (T), cytosine (C) or guanine (G)) of a genome sequence.
Preimplantation genetic diagnosis (PGD) was developed in the 1980's as an option for those couples who were at risk of having a child affected by a monogenic disease or a specific chromosomal disorder and who wanted to avoid the possibility of requiring voluntary termination of pregnancy.
The PGD consists of performing a biopsy of one or more cells of the embryos generated during an *in vitro* fertilisation cycle when the embryos are between 3 to 5 days old, to use said material in drawing up a genetic diagnosis. Therefore, only those embryos diagnosed as unaffected by a specific genetic alteration are transferred in order to result in a healthy child.
It should be noted that PGD has two features that differentiate it from the genetic diagnosis applied to any other field. On one hand, the response time must be much shorter, since in many cases it is necessary to obtain results in less than 24 hours to enable embryos to be transferred in the same cycle. Thus, for example, an embryo biopsied on day 3 must be transferred or vitrified on day 5 or 6. On the other hand, each couple normally produces an average of 6 to 10 embryos, such that the cost of the analysis must be low to be able to analyse all the embryos and that this does not entail a substantial increase in the current cost of an *in vitro* reproduction cycle.
Due to the above, PGD is carried out using multiple and varied techniques, depending on the nature of the alteration studied. Traditionally, for monogenic diseases, methods based on polymerase chain reaction (PCR) and fragment analysis and, more recently, systems like Karyomapping based on the use of microarrays applied to SNP detection (for example, as described in document ES2360085T3), were used. Despite being very different, all methods share common features:

Firstly, they require prior amplification. In this case very little starting genetic material, often coming from a single cell, is used, such that it is necessary to amplify it. To do so, a method known as MDA (Multiple Displacement Amplification) that amplifies the genetic material thanks to the Phi29 polymerase at a constant temperature is traditionally used. This method produces long DNA fragments with a low error rate.

Secondly, they have a high Allele-Dropout (ADO) rate. ADO is a common artefact in this type of analysis, about 5% of the analyses have it, which consists of the preferential amplification of one of the alleles. For this reason, if an analysis does not detect a mutation when carried out, it is possible that it is due to the fact that the mutated allele has not been amplified.

Thirdly, an indirect analysis is carried out. As a consequence of the above, an indirect analysis of the mutation is always carried out. This analysis consists of studying a series of polymorphisms around the mutation, generally STR or Short Tandem Repeats, and determining whether the polymorphisms present in the embryo are those associated with the pathological allele or the healthy allele, this is, a linkage analysis is carried out.

Fourthly, they require a prior informativity study. In order to determine which polymorphisms segregate with the healthy allele and which ones with the pathological allele, it is necessary to carry out a prior informativity study. This study consists of analysing 5 to 10 STRs close to the mutation, located on both sides, in the couple and in other family members. Ideally, a child in common with the mutation, or parents of the couple. Thus, for example, if it can be established that a specific number of repetitions of a specific STR is always present in relatives with the pathogenic mutation, said polymorphism may be associated with the pathological allele and those embryos that possess it may be discarded.

Lastly, the direct analysis of the mutation is not always possible. Whenever possible, the direct analysis of the mutation of the embryos is generally carried out through mini sequencing. However, this is only possible if it is a point mutation. With this kind of technique, it is not possible to detect other types of alterations, such as deletions. In turn, Karyomapping is not able to detect the direct mutation in any case.

Recently, massive sequencing (NGS) has been incorporated as a technique to the PGD, as described in documents US20140274741, WO2014082032, US20150038337 or EP2947156. Nevertheless, only its application to the detection of aneuploidies (PGD-A) is described in the state of the art.

Although technology has evolved in the improvement of these PGD techniques, there are currently certain limitations on the analysis technique. The main techniques and their limitations on PGD are:
Microarray CGH (Comparative Genomic Hybridisation). This technique is used for detecting aneuploidies (PGD-A) and unbalanced alterations when one of the parents is a carrier of a balanced alteration. The main limitations of the technique are:

It is an expensive technique, such that many couples who would truly benefit from it, for example, older mothers since a high number of their oocytes would be aneuploidies, would not be able to choose them.

A PCR-based amplification method is used, which has a much higher percentage of ADO than the MDA-based methods, which hinders the integration with techniques for the study of monogenic diseases, as indicated above.

It requires specific equipment, such as a microarray scanner.

It does not allow for the analysis of mutations, such that it cannot be used to select embryos free from genetic pathology when the parents are carriers.

It does not allow distinction between normal embryos and those that carry a balanced translocation.

It does not allow identification of those embryos that carry numerical anomalies in mosaic.

Mini sequencing. It is used for directly detecting a specific mutation in the study of monogenic diseases in embryos (PGD-M). The main limitations are:

It requires the design of specific primers in a very specific region, which can hinder the analysis.

It requires previous amplification through MDA, making it difficult to combine with techniques for the screening of aneuploidies.

Prior knowledge of the mutation to be analysed and a long set-up process is needed.

It requires a capillary sequencer.

It does not enable structural anomalies of any kind to be detected.

It is only useful in the case of point mutations.

Fragment analysis. It is used alone or in conjunction with the above, to carry out the indirect study of the pathologies, such that the limitations are the same as those in the case of mini-sequencing, in addition to,

it requires the design of specific primers fluorescently labelled to design the prior informativity study.
Sometimes, the informativity study can be prolonged due to the difficulty of finding informative STRs.

*Karyomapping.* It is a technique that analyses thousands of polymorphisms by microarray, combining the analysis of aneuploidies and the study of monogenic diseases. The great advantage is that the same microarray is used to analyse different pathologies. Its limitations are the following:

Although it detects aneuploidies, it is not able to detect mitotic errors, which means that it is not capable of determining the presence of mosaicism.
It requires samples in trio (father, mother and child previously affected) to determine the segregation of the alleles.

This is because it only carries out an indirect study.

In no case is it possible to detect the mutation itself, such that the risk of recombination is never excluded.

It is expensive.

The protocol is long, and thus cannot be used for cycles with fresh transfer.

Yan et al. (2015; PNAS, vol. 112, no. 52, pages 15964-15969) discloses an NGS-based PGD procedure that can simultaneously detect a single-gene disorder and aneuploidy and is capable of linkage analysis.

ES2360085T3 discloses that chromosomal analysis by molecular karyotyping (for example, for the detection of trisomy) can be carried out using an analysis of biallelic markers of the whole genome (e.g., biallelic single nucleotide polymorphisms (SNP)) that are distributed by the genome, and that can be easily detected using existing technologies. This discovery is unexpected for several reasons, but mainly because a priori it would be assumed that a biallelic marker (which only provides binary information at a given position on the chromosome) could not positively identify the presence of three or more different chromosomes.

Nevertheless, this document carries out a high-density analysis of nearby adjacent SNPs and is able to positively identify, among others, the presence of two chromosomes derived from a parent and based on well-established assumptions about the frequency and spacing of recombination events between parent chromosomes during meiosis, this will enable accurate detection of trisomy. Furthermore, the parental origin of the error is identified in each case, which is not possible by some karyotyping methods.

However, it has not yet been possible to successfully establish a quick, effective and economical method that enables PGD-A and PGD-M to be combined with a single biopsy. Therefore, progress in the technique accompanies the improvement of the PGD-A and PGD-M tools with a single biopsy by massive sequencing, a main line in the development of the present project.

## DESCRIPTION OF THE INVENTION

An object of the present invention is a method that combines PGD-A and PGD-M techniques with a single biopsy by massive sequencing. This object is achieved with the method of claim 1. Particular embodiments of the method of the invention are shown in the dependent claims. In other aspects, the kit including an electronic device that executes the method of the invention, as well as the software product that contains the executable instructions for carrying out the method of the present invention is claimed.

For the combination of both techniques, first, a PGD-A library is prepared. Any available commercial kit can be used in the preparation of this library, such as, for example, *Ion Reproseq (ThermoFisher), PicoPlex (Rubicon Genomics), Veriseq (Illumina) and Repli-G (Qiagen).*

Subsequently, an aliquot of that amplified DNA is taken, and a method to enrich the SNPs of interest is applied. Preferably, this method is based on multiplex amplification, but capture, simple PCR, or any other method can also be used. After the amplification of the regions of interest, the libraries are then prepared, by adding the necessary adapters and barcodes. Thus, the library for PGD-M will be obtained.

Finally, after the quantification of both libraries, they are combined in specific proportions, and the standard sequencing protocol for the chosen platform is continued. Since different library preparation methods are used for both processes, each of them with their barcode, the sequencer yields the results of PGD-A and PGD-M separately. Thus, the analysis is carried out independently for PGD-A, using the appropriate solution according to the selected library preparation method and sequencer, whereas the PGD-M analysis is carried out by phasing SNPs, as described later.

This protocol is faster than other technologies such as Karyomapping (ES2360085T3) because it only adds four hours (preparation time of the PGD-M library) to the overall PGD-A process by massive sequencing. In this manner, if, for example, the combination of *Ion Rerproseq* for PGD-A together with the method of the invention is used, the whole process can be carried out in less than twelve hours, being, therefore, an ideal method for performing biopsy in D-3 and transfer in D-5, but also for biopsy in D-5 and transfer in D-6 and, obviously, for cycles with deferred transfer since in the protocol there are multiple steps where it can be stopped.

This method is also more economical than the methods based on karyotyping or karyomapping (ES2360085T3) since the price depends on the cost of library preparation for PGD-A. Furthermore, it is adaptable to the number of samples that are to be analysed, whereas in karyomapping a slide of 12 arrays is used, such that the number of samples must be a multiple of 12 in order to maximise results.

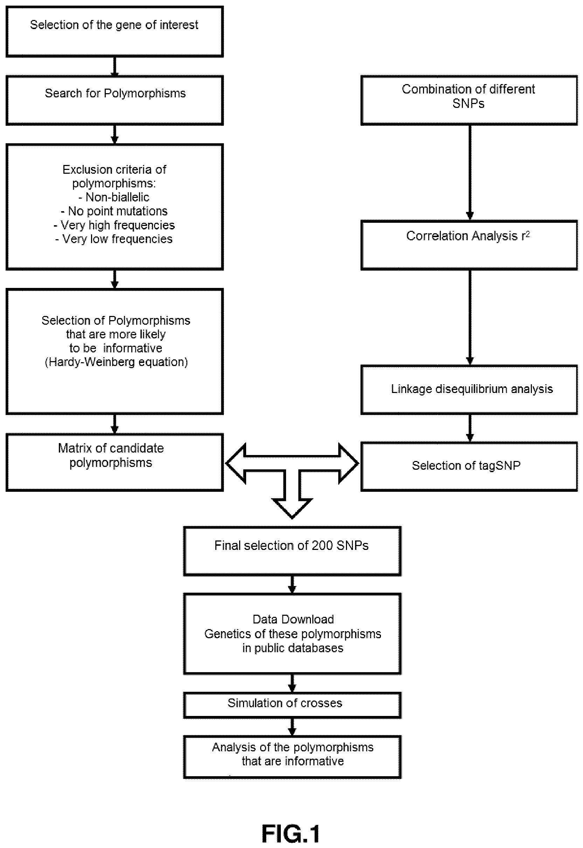

For example, a library for PGD-A can be prepared using Ion Reproseq. If the couple has, for example, 8 embryos, 8 different libraries with the corresponding barcodes will have been prepared, from 1 to 8. Later, an aliquot is taken and the corresponding polymorphisms are amplified. If the couple is a carrier, for example, of mutations in the SMN1 gene, a kit that amplifies certain polymorphisms around that mutation will be used. After the amplification, the library will be prepared using the corresponding method. One method could be amplification by means of multiplex PCR using Ampliseq, with its corresponding library preparation kit, and adding different barcodes to the previous ones (for example, from 9 to 16). Both libraries are quantified, and they are mixed in a 3:1 ratio, that is to say, three times more PGD-A library than PGD-M library. After quantification, if the selected sequencing method is, for example, Ion PGM, the preparation of the sequencing and the sequencing itself is then carried out, using the standard protocol of the equipment manufacturer. Once the sequencing is finished, the sequencer will yield 16 files, 8 for the PGD-A library and 8 for PGD-M. The files for PGD-A will be analysed using the equipment manufacturer's software, or any other solution. For the PGD-M analysis, SNP phasing will be carried out using the software proposed in the present invention. FIG. 1 shows an outline of the complete process.

Thanks to the method described, it is possible to combine PGD-A and PGD-M techniques by means of massive sequencing. Furthermore, it is considerably faster than technologies based on karyotyping or karyomapping, in addition to being more economical.

Throughout the description and the claims, the word "comprises" and its variants are not intended to exclude other technical features, additives, components or steps. For those skilled in the art, other objects, advantages and features of the invention may be inferred from both the invention and the embodiments of the invention. The following examples and drawings are provided by way of illustration and do not seek to limit the present invention. Furthermore, the invention covers all possible combinations of particular and preferred embodiments indicated herein.

## BRIEF DESCRIPTION OF THE DRAWINGS

What follows is a very brief description of a series of drawings that aid in better understanding the invention, and which are expressly related to an embodiment of said invention that are presented by way of a non-limiting example of the same.

FIG.1 shows an outline of the method object of the present invention. Firstly, the gene with the mutation to be discarded in the embryos is analysed and a surrounding region is selected (typically, 4 Mb). Those polymorphisms that do not meet any exclusion criteria are selected, and those most likely to be informative are searched. Furthermore, those polymorphisms that can be tag by correlation and linkage disequilibrium analysis are identified. With both, a final panel design is made, and *in silico* validation is performed by simulating cross-links among a multitude of subjects whose genomes are available in public databases.

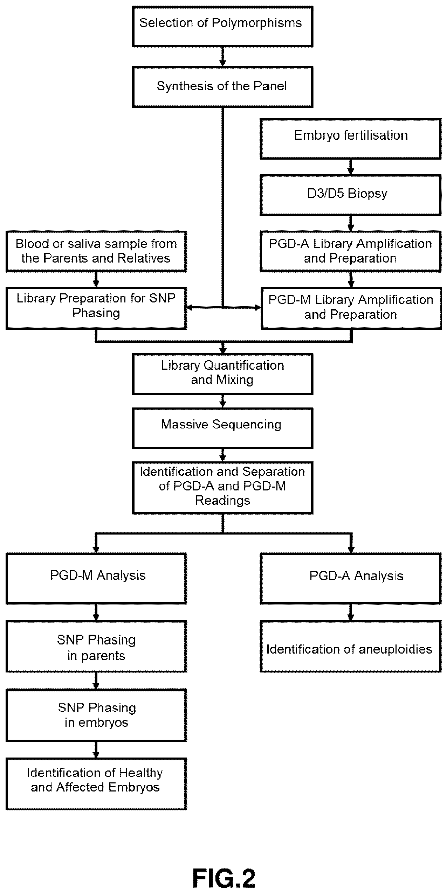

FIG.2 shows an outline of the whole process, including the method of the invention, for a first example of use, starting with the design and synthesis of the panel that will then be used to amplify the library for PGD-M. On the one hand, patients and other relatives are analysed to determine the distribution of polymorphisms in the different alleles. As for the samples of the embryos, after the biopsy, the library for PGD-A is prepared, and with an aliquot thereof the library for PGD-M is prepared. All libraries are quantified and mixed, and then sequencing is carried out. Lastly, the bioinformatic analysis of aneuploidies and monogenic disease is independently performed.

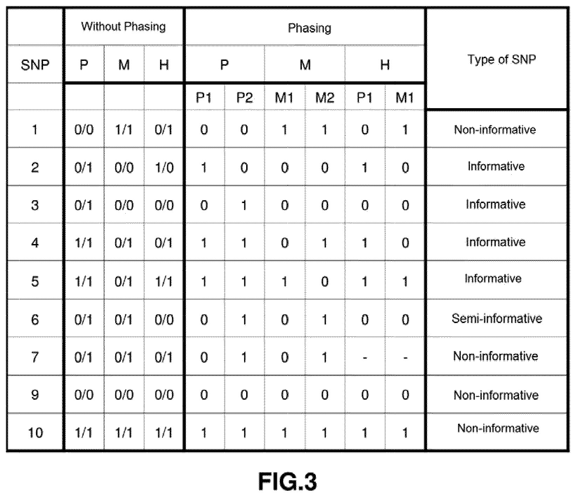

FIG.3 shows an example of SNP phasing, in the case of a trio formed by the couple and an affected child. The SNPs are shown before and after phasing, with the distribution thereof in the alleles. Furthermore, it indicates which ones are informative (they give information about the phase) and which ones are not.

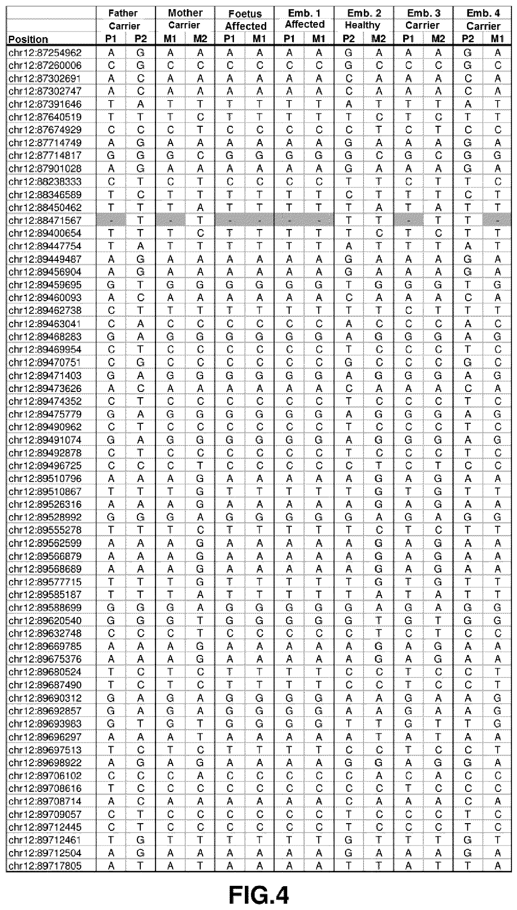

FIG.4 shows an example of phasing of SNPs with analysis of results in embryos. In short, only those SNPs that are informative are shown. In this case, the couple carries mutations in heterozygosis in the *CEP290* gene, which causes Meckel syndrome. As this syndrome is a prenatal lethal disorder, DNA from a previous foetus was used for phasing. In this case, the panel was designed including the direct analysis of the mutation, which is the shaded area.

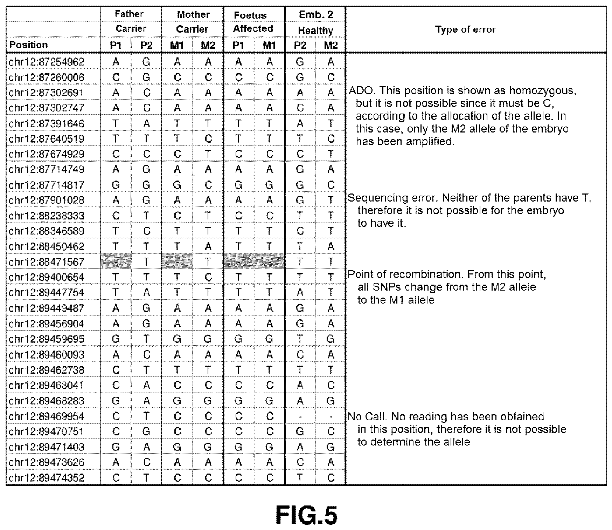

FIG.5 shows the result of SNP phasing in an embryo, where different problems and artefacts arise, along with the description thereof. The developed phasing algorithm enables these errors to be detected.

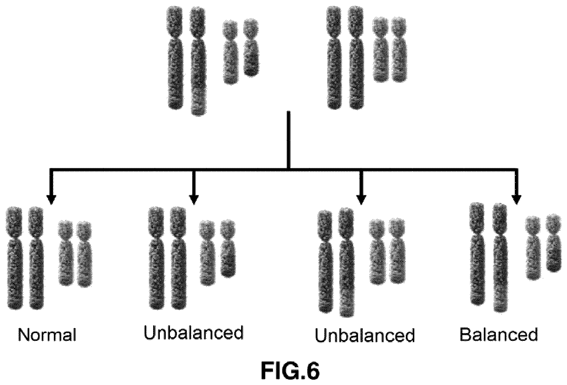

FIG.6 shows an example of a couple with a balanced translocation, and the embryos that can be produced as a result. 50% of these embryos can inherit an unbalanced alteration with serious consequences (from repeat abortions to children with mental retardation and dysmorphic features). 25% of the embryos will be normal, and the other 25% will have balanced alteration like one of the parents.

## DESCRIPTION OF A DETAILED EMBODIMENT OF THE INVENTION

The method for the study of embryo mutations in *in vitro* reproduction processes object of the present invention can be divided into three sequential and differentiated processes. The method of the invention combines several tagSNP selection techniques, since it calculates the linkage disequilibrium correlations for the block of interest (by default, 4 Mb around the mutation, 1 Mb is equal to one million nucleotides). The SNPs present in this region, in turn, will be considered as in a block-free approach, such that all the correlations between SNPs are calculated and taken into account in the selection of the tagSNPs. In this way, the present invention selects polymorphisms that are highly likely to be informative, considering the allele frequencies of the same within the target population and whether or not they are part of the same haploblock (set of SNPs that are inherited together). A tagSNP is the SNP that is considered representative for the entire haploblock, that is to say, that if the tagSNP is in heterozygosis, for example, all the SNPs belonging to that same haploblock will be in heterozygosis. A tagSNP avoids having to analyse all the polymorphisms because by knowing how it behaves, it is possible to deduce how the rest of the polymorphs of the haploblock behave.

Therefore, the objective of the method of the invention (FIG.1) focuses on obtaining a minimum tagSNP panel with maximum informative capacity, simplifying the subsequent analysis and interpretation of the results of an informativity analysis.

More specifically, the method of the invention is divided into two basic processes and a third validation process. The first two processes do not have to necessarily be in this order:

**(i)** A first SNP selection process:

a. In this process, the values of the n candidate SNPs ($t_1$ ... $t_k$) of each subject x, in a chromosomal region of interest and specifically extracted for the studied population, are taken as an input. SNPs that are biallelic are selected, such that subjects can be represented as length haplotypes m formed by binary strings {1,0}, being 1|0 and 0|1 the values for heterozygous SNPs and 010 and 111 the values for the homozygotes. This is done in the entire chromosomal region of interest that is defined as any position that is 2 Mb (it is a default value that can be modified) upstream and 2 Mb downstream of the gene/mutation that is to be studied.

b. Subsequently, the n candidate SNPs of the region are analysed and those that meet any of the following conditions are excluded:

i. SNPs with more than one alternative allele (non-biallelic SNPs)

ii. SNPs whose alleles are different from the change of a single nucleotide (indels, changes of polynucleotide pattern, among others)

iii. SNPs that are homozygous in at least 99% of the population of interest

iv. Uncommon SNPs, that is, whose minor allele frequency is less than 1%

c. The next step is to maximise the situation in which one of the parents has the value of an SNP in a heterozygous state, while in the other parent it is presented as a homozygote, that is, it is informative. This is achieved through the maximisation of the value of two functions above a certain threshold value:

$$\text{MaxP: } p-(3p2) + (4p3) - (2p4)$$

**HET rate:** 2pq

wherein *p* and *q,* respectively, are the allele frequencies of the reference and alternative alleles for each SNP. These are the equations of the Hardy-Weinberg equilibrium and its derivative.

d. The output of this algorithm will be a panel of *z* optimised SNPs for both values in the form of *matrix M* whose columns correspond to the subjects of the population and the rows to the values of each SNP for each subject.

**(ii)** A second SNPs selection process:

a. Through an exhaustive search, all the SNP combinations are evaluated to obtain a minimum set *t* of tagSNPs from the matrix *M* obtained in point (i).

b. First, the SNPs of the matrix *M* of the block-region are organised in groups of high correlation based on the pairwise $r^2$ criterion. To do this, the pairwise $r^2$ value is calculated from the allele frequency calculated for the matrix *M.* In this way SNPs from different groups will present low correlation, such that two SNPs will belong to the same group only when the pairwise $r^2$ therebetween exceeds a certain threshold value (set by the user).

c. After this, the selection of tagSNPs within each group is made based on the LD criterion, starting with k=1 SNPs and studying all possible k-combinations, organising the SNPs within each group.

d. Assuming two SNPs whose frequencies are p (most frequent allele) and q = *1-p*, the following equations are used:

$$D = pAB - pApB \qquad D' = D/Dmax \quad \begin{cases} D > 0 \rightarrow Dmax = \min(pApb, papB) \\[2em] D < 0 \rightarrow Dmax = \max - pApB, -papb \end{cases}$$

Being pA and *pB* the observed probability of the allele *p* for SNP1 and SNP2, respectively, and *pa* and *pb* the probability for the minor allele. Lastly, *pAB* is the combined probability of the pair *pq.* By using these equations, SNPs whose functional ranges exceed a certain threshold value will be considered tagSNPs.

The organisation of the SNPs within each group based on the LD criterion enables a selection to be made based on the functional range, ensuring that the first solution found is an optimal solution and enabling the computing time to be considerably reduced.

Finally, if a SNP does not exceed the $r^2$ or LD thresholds it will be considered in one group only and taken as tagSNP by itself.

**(iii)** A third validation process consisting of an in-silico validation of the tagSNP panel obtained. For this purpose and using the *1000Genomes db* database, subjects are randomly chosen to perform multiple crosses. After this, the number of informative tagSNPs of each crossing is counted and the average is provided as informative data of the informative power of the panel.

As indicated, **FIG.2** shows an outline of the method of the invention, where firstly, the gene with the mutation to be discarded in the embryos is analysed and a surrounding region is selected (typically, 4 Mb). Those polymorphisms that do not meet any exclusion criteria are selected, and those most likely to be informative are searched. Furthermore, those polymorphisms that can be a tag by correlation and linkage disequilibrium analysis are identified. With both, a final panel design is made, and *in silico* validation is performed by simulating cross-links among a multitude of subjects whose genomes are stored in public databases.

**Example 1. Diagnosis of aneuploidies**

In **FIG.2** the complete outline for use in the diagnosis of aneuploidies is shown. It is considered a European population couple wherein a member of the couple is a carrier of the autosomal dominant pathogenic variant VHLc.233A>G p.(Asn78Ser) (chromosomal position *chr13:10183764*) causing a condition known as Von Hippel-Lindau syndrome, which has an autosomal dominant mode of inheritance.
The input of the software will be the 69473 SNPs contained in the block-region *chr13:9181319-11681319.* The output of this algorithm will be a matrix M of 1625 candidate SNPs, which will act as input for the SNP selection algorithm, whose output will be a panel of 283 tagSNPs. In the validation phase, it was found that on average 49% of tagSNPs in the panel were informative.
**Wet laboratory protocol.** Once selected the polymorphisms that are to be sequenced, positions are entered into the corresponding enrichment platform. Preferably, Ion Ampliseq. This platform designs the primers needed to capture the regions. In the IVF laboratory, produced embryos are biopsied when they reach the blastocyst stage. The biopsy is placed in a PCR tube and sent to the laboratory. At the laboratory, the DNA is amplified using, for example, Ion Reproseq, so that, in addition to the amplification, the library is made for PGD-A. Following the appropriate protocol, all the regions designed with Ampliseq in the previously amplified material are amplified, and the library for PGD-M is produced. Subsequently, massive sequencing is carried out. It is important to keep in mind that in order to be able to simultaneously sequence multiple samples, it is necessary to mark the samples with a molecular barcode. Special care must be taken in that the barcodes do not coincide between the samples.
**Data analysis.** Once the sequencing is finished, a series of files with readings for the whole embryo (PGD-A) and other files with the detected polymorphisms (PGD-M) are obtained. A bioinformatic analysis must be carried out with these files. With the first files, the aneuploidies are determined using the most appropriate software according to the platform. With the second files, the segregation pattern of each polymorphism is determined for the PGD-M analysis. Firstly, the obtained readings are aligned to the reference genome, and polymorphisms are identified in each and every sample, including patients, relatives used as a reference and embryos. In the case of relatives, the simplest situation is that in which we have the couple and an affected child. For SNP phasing, it is necessary to determine which polymorphisms are shared in the trio and in this way, to find out which ones segregate with the healthy allele and which ones segregate with the pathogenic allele. Since biallelic SNPs have been selected, each of the samples can be 0/0, 0/1, 1/1 if they are homozygous for the wild SNP, heterozygous or homozygous for the alternative SNP, respectively. The number 0 indicates that it is the reference SNP (whatever it is) while 1 indicates that it is the alternative SNP. This is true for all chromosomes, except sex chromosomes, in which women can be homozygous or heterozygous, while men are always hemizygotes (0 or 1). With this data, the SNP phasing is then carried out. To do so, those SNPs that are informative in the couple are analysed. Informative SNPs are those in which one of the patients is heterozygous (0/1) and the other homozygous (0/0 or 1/1). The polymorphism that is used for phasing is that which is different in the heterozygous subject. For example, if we have a subject 0/1 and the other 1/1, the polymorphism that we will use for phasing is 0. By comparing one or more subjects in the family, it is arbitrarily determined which allele each one belongs to. An example of SNP phasing would be the following:

Considering the situation in which there is a couple with a child, each with their alleles. It is considered that the father has the alleles P1 and P2, the mother M1 and M2. Logically, the child will have inherited one allele from each, for example, P1 of the father and M1 of the mother. If when analysing polymorphisms, the result is that the father is 0/1, the mother 1/1 and the child 0/1, it will be determined that the polymorphism 0 belongs to the P1 allele, which is shared between father and child, since both have said polymorphism. In another case, the father may be 0/1, the mother 1/1 and the child 1/1. In this case, the polymorphism 0 must necessarily belong to the P2 allele of the father, since it is not shared between father and child. We process all the polymorphisms that are informative for the mother in a similar manner.

Once the haplotypes of the parents are determined, the analysis of the embryos is carried out. To do so, the informative SNP in each one of the embryos is identified. For example, if polymorphism 0 belongs to the P1 allele, if an embryo has said polymorphism, it means that it has said haplotype. Indeed, the informative SNPs in the different embryos are identified, thus determining the haplotype of each of them.

An example of the SNP phasing process is shown in **FIG.3,** wherein it is specified, moreover, whether the SNPs are informative, non-informative or semi-informative. An informative SNP is one that complies with the above, that is to say, it is heterozygous in one of the parents, and homozygous in the other; A non-informative SNP can occur when both parents are homozygous, or when both are heterozygous and the child is heterozygous as well; a semi-informative SNP occurs when both parents are heterozygous and the child is homozygous. This classification can be extended to other family combinations.

Once SNP phasing has been carried out with relatives, the pattern of polymorphisms must be compared with the sequenced embryos, and in this way, it will be determined which embryos are carriers and which are normal. In **FIG.4** an SNP phasing example is shown that includes the analysis of the embryos.

The SNP phasing algorithm is, moreover, able to identify the different possible sources of error and alert the analyst so that he/she can weigh and analyse them. There are different sources of error. In **FIG.5** the result is shown for an embryo similar to **FIG.4,** but where the different sources of error are identified and described:

For example, there can be an allele dropout phenomenon. This phenomenon implies that, in an embryo, only one of the alleles is amplified. In this way, when sequencing, it can be interpreted that an embryo is homozygous for a polymorphism, when in reality it is heterozygous. For example, an embryo can be 0/1 but have suffered an ADO phenomenon during the amplification and thus be shown as 1/1 in the sequencing. This error can lead to a misinterpretation of the results, mistakenly assigning the allele of the embryos. In order to avoid it, it is necessary to distinguish those polymorphisms that, in addition to being informative, are *key*. The key polymorphisms are those that, in addition to being informative, are heterozygous in the embryo. For example, if there is a father 0/1 and a mother 1/1, and the embryo is 0/1, the polymorphism is key because it is informative and heterozygous. On the contrary, if the embryo is 1/1, the polymorphism is only informative. In this last case it is not possible to determine whether the embryo is really 1/1, or if there has been allele dropout and only one of the alleles 1 is seen.

Another source of error is due to what is called *No Call.* It occurs when no signal is obtained for any of the alleles for a given polymorphism, so it is impossible to know even one of them.

Finally, another source that can lead to confusion is recombination. Recombination is when the alleles in one of the parents are exchanged, and this is reflected in the embryo. For example, if 100 polymorphisms in an embryo are analysed, it may happen that part of them (for example, 60) belong to the P1 allele and the remainder belong to the P2 allele. In order to identify recombination, the change of allele P1 to P2 must be sequential. That is, for example, that the first 60 polymorphisms belong to the P1 allele and the following belong to the P2 allele. If the exchange of polymorphisms occurred more or less randomly, that would mean that it is a sequencing error, since statistically it cannot be the case that an embryo has more than two recombinations in a space as small as the fragment analysed (4 Mb).

It can also happen that there are sequencing errors or artefacts. These artefacts can be easily identified because they seem to be recombinations or allele dropouts, but they happen sporadically in one or very few positions.

Lastly, in order to be able to carry out the allocation of alleles unequivocally, it is required that there be at least three informative polymorphisms and that they also be key on each side of the mutation, consecutively, in addition to at least 3 other non-key polymorphisms.

## Example 2. Identification of triploid embryos

Triploid embryos are a major problem in any IVF cycle. They account for 15% of miscarriages due to chromosomal abnormalities. Triploid embryos should always be discarded from any *in vitro* fertilisation cycle, but it is difficult to identify them because there are no differences in embryo quality with respect to normal embryos. Sometimes, it is possible to distinguish them because in D+1 three pronuclei are observed, but it is not always possible. The triploid embryos may be of a dyspermic origin (in cases of IVF) or be originated by an oocyte failure when the second polar corpuscle is not extruded.
Triploid embryos cannot be identified by ordinary PGD-A techniques, despite being a numerical anomaly. Sometimes, through visual inspection, it is possible to detect embryos 46, XXY when observing an abnormal distribution of the readings of the sex chromosomes, but it is not always possible and requires trained personnel.
The method herein described can be used to identify this type of embryo. Informative polymorphisms can be selected along the genome and it can be determined whether they are triploids by analysing the polymorphisms present and the frequency thereof. Normally, a polymorphism in heterozygosis should be found in a proportion of around 0.5, since half of the readings will correspond to one allele and half to another. A triploid embryo has three alleles, so this proportion will be diverted. Thus, the result can be three polymorphisms for the same position (if they are multiallelic) or two polymorphs but one of them with frequency over 33% and the other over 66%. If all polymorphisms with sufficient readings follow this pattern throughout the entire genome, this means that the embryo is triploid.

## Example 3. Identification of embryos with balanced translocations

Sometimes, some couples decide to undergo *in vitro* fertilisation cycles because one of them is a carrier of a balanced translocation. In these cases, these parents have a high reproductive risk, since 50% of their embryos will have an unbalanced translocation as a result of inheriting one of the altered chromosomes. Furthermore, there will be a 25% chance of producing completely normal embryos, and a 25% chance of producing embryos with the balanced alteration. **FIG.6** shows an outline of the possible embryos produced. Current techniques enable those embryos with unbalanced alterations to be distinguished, most of the time by simply using PGD-A. Nevertheless, it is not possible to differentiate those embryos with the balanced alteration from those that are completely normal, since there are no changes in copy number. Through the present development, it is possible to map the entire chromosome by different polymorphisms and, by studying the distribution of these polymorphisms in the unbalanced embryos, determine which are present in the altered chromosome and which are in the normal one. In this manner, it will be possible to know whether the embryos without changes in copy number have received from their parent the normal chromosome or the altered one. This study is possible thanks to the combination of PGD-A and PGD-M.

## Claims

1. A method for the preimplementation genetic diagnosis of embryo mutations in *in vitro* reproduction processes with the particular feature that it combines a single biopsy with Next Generation Sequencing (NGS), the detection techniques of Aneuploidy (PGD-A) and the study of monogenic embryonic diseases (PGD-M) and **characterised in that** it comprises the processes of:

   a first SNP selection process wherein:

   (i) the values of some n candidate SNPs ($t_1 \ldots t_k$) of each subject x, in a chromosomal region of interest and specifically extracted for a study population, are taken as an input; and wherein this process is configured to maximise the situation in which one of the parents has the value of an SNP in a heterozygous state, while the other parent has the value of an SNP in a homozygote state, and to obtain a panel of z optimised SNPs for both maximised values in the form of matrix M whose columns correspond to the subjects of the population and the rows to the values of each SNP for each subject;

(ii) the process comprises the selection of those SNPs that are biallelic, wherein subjects can be represented as length haplotypes m formed by binary strings {1,0}, wherein 1|0 and 0|1 are the values for heterozygous SNPs and 010 and 111 are the values for the homozygotes SNPs; wherein this selection is made throughout the chromosomal region of interest;

(iii) the process comprises a stage of analysing the n candidate SNPs in the region and excluding the SNPs that meet any of the following conditions: SNPs with more than one alternative allele (non-biallelic SNPs); SNPs whose alleles are different from the change of a single nucleotide; SNPs that are homozygous in at least 99% of the population of interest; and uncommon SNPs, wherein the minor allele frequency is less than 1%; and

(iv) the process comprises a stage of maximising the situation in which one of the parents has the value of a SNP in a heterozygous state, while the other parent has the value of the SNP in a homozygote state, wherein is informative through the maximisation of the value of two functions above a certain threshold value:

$$\textbf{MaxP}: p-(3p2) + (4p3) - (2p4)$$

**HET** rate: 2pq

wherein p and q are, respectively, the allele frequencies of the reference and alternative alleles for each SNP;

a second SNP selection process wherein:

(v) all the SNP combinations are evaluated to obtain a minimum set t of tagSNPs from the matrix M obtained in the first SNP selection process; and an in-silico validation process of the tagSNP panel obtained in the second process;

(vi) the process comprises, firstly, that the SNPs of the matrix M of the block-region are organised in groups of high correlation based on the pairwise $r^2$ criterion; wherein the pairwise $r^2$ value is calculated from the allele frequency calculated for the matrix M;

(vii) wherein the SNPs of different groups will present low correlation, wherein two SNPs will belong to the same group only when the pairwise r2 therebetween exceeds a certain threshold value set by the user; and

(viii) wherein the selection of tagSNPs within each group is made based on the detection limit (LD) criterion, starting with k = 1 SNPs and studying all possible k-combinations, organising the SNPs within each group; and

a third validation process, wherein a genomic database is used where subjects are randomly chosen to perform 300 crosses, after which the number of tagSNPs that were informative of each crossing is counted and the average is provided as informative data of the informative power.

2. The method according to claim 1, wherein the chromosomal region of interest is defined as any position that is located two megabases above and two megabases below the gene or mutation under study.

3. The method according to any one of claims 1 to 2, wherein if a SNP does not exceed the $r^2$ or LD thresholds it will be considered in one group only and taken as tagSNP by itself.

4. A kit for the preimplementation genetic diagnosis of embryo mutations in *in vitro* reproduction processes, **characterised in that** it comprises, at least one electronic device with a processor or processors and a memory, wherein the memory stores instructions that when executed by the processor or processors cause the electronic device to execute the method according to any one of claims 1 to 3.

5. A computer program product with instructions configured to be executed by one or more processors that make the electronic device of the kit of claim 4 carry out the method according to any one of claims 1 to 3.

**Patentansprüche**

1. Verfahren zur genetischen Präimplantationsdiagnose von Embryomutationen in *In-vitro*-Reproduktionsprozessen mit der Besonderheit, dass es eine einzige Biopsie mit Next Generation Sequencing (NGS), die Nachweistechniken der Aneuploidie (PGD-A) und die Untersuchung monogener embryonaler Erkrankungen (PGD-M) kombiniert, und **dadurch gekennzeichnet, dass** es folgende Prozesse umfasst:

einen ersten SNP-Auswahlprozess, wobei:

(i) die Werte einiger *n* Kandidaten-SNP ($t_1$ ... $t_k$) jedes Subjekts *x*, in einer Chromosomenregion von Interesse und speziell für eine Studienpopulation extrahiert, als Eingabe genommen werden; und wobei dieser Prozess so konfiguriert ist, dass die Situation maximiert wird, in der ein Elternteil den Wert eines SNP in einem heterozygoten Zustand aufweist, während der andere Elternteil den Wert eines SNP in einem homozygoten Zustand aufweist, und dass ein Panel von *z* optimierten SNP für beide maximierten Werte in Form einer Matrix *M* erhalten wird, deren Spalten den Subjekten der Population und deren Zeilen den Werten jedes SNP für jedes Subjekt entsprechen;

(ii) der Prozess die Auswahl jener SNP umfasst, die biallelisch sind, wobei die Subjekte als durch binäre Zeichenfolgen {1,0} gebildete Längenhaplotypen *m* dargestellt werden können, wobei 1|0 und 0|1 die Werte für heterozygote SNP und 0|0 und 1|1 die Werte für die homozygoten SNP sind; wobei diese Auswahl im gesamten Chromosomenbereich von Interesse erfolgt;

(iii) der Prozess eine Phase der Analyse der *n* Kandidaten-SNP in der Region und den Ausschluss der SNP, die eine der folgenden Bedingungen erfüllen, umfasst: SNP mit mehr als einem alternativen Allel (nicht biallelische SNP); SNP, deren Allele sich durch die Veränderung eines einzelnen Nukleotids unterscheiden; SNP, die in mindestens 99 % der Population von Interesse homozygot sind; und seltene SNP, bei denen die Frequenz seltener Allele weniger als 1 % beträgt; und

(iv) das Verfahren eine Phase der Maximierung der Situation, in der ein Elternteil den Wert eines SNP in einem heterozygoten Zustand aufweist, während der andere Elternteil den Wert des SNP in einem homozygoten Zustand aufweist, umfasst, wobei es durch die Maximierung des Werts zweier Funktionen über einem bestimmten Schwellenwert informativ ist:

$$\text{MaxP: } p-(3p2) + (4p3) - (2p4)$$

**HET-Rate:** 2pq

wobei p und q jeweils die Allelfrequenzen der Referenz bzw. alternative Allele für jeden SNP sind;

einen zweiten SNP-Auswahlprozess, wobei:

(v) alle SNP-Kombinationen ausgewertet werden, um einen Mindestsatz *t* von tagSNP aus der im ersten SNP-Auswahlprozess erhaltenen Matrix *M* und einen *In-silico*-Validierungsprozess des im zweiten Prozess erhaltenen tagSNP-Panels zu erhalten;

(vi) das Verfahren zunächst umfasst, dass die SNP der Matrix *M* der Blockregion in Gruppen hoher Korrelation basierend auf dem paarweisen $r^2$-Kriterium organisiert sind; wobei der paarweise $r^2$-Wert aus der für die Matrix *M* berechneten Allelfrequenz berechnet wurde;

(vii) die SNP verschiedener Gruppen eine geringe Korrelation präsentieren, wobei zwei SNP nur dann zur selben Gruppe gehören, wenn der paarweise $r^2$ zwischen ihnen einen bestimmten, vom Benutzer festgelegten Schwellenwert überschreitet; und

(viii) die Auswahl der tagSNP innerhalb jeder Gruppe auf der Grundlage des Kriteriums der Nachweisgrenze (LD) erfolgt, wobei mit k = 1 SNP begonnen wird und alle möglichen k-Kombinationen untersucht werden, wobei die SNP innerhalb jeder Gruppe organisiert werden; und

einen dritten Validierungsprozess, bei dem eine Genomdatenbank verwendet wird, in der Subjekte nach dem Zufallsprinzip ausgewählt werden, um 300 Kreuzungen durchzuführen, und im Anschluss daran die Anzahl der tagSNP, die für jede Kreuzung informativ waren, gezählt wird und der Durchschnitt als informative Angabe der Aussagekraft bereitgestellt wird.

2. Verfahren nach Anspruch 1, wobei die Chromosomenregion von Interesse als jede Position, die sich zwei Megabasen über und zwei Megabasen unter dem untersuchten Gen oder der untersuchten Mutation befindet, definiert ist.

3. Verfahren gemäß einem der Ansprüche 1 bis 2, wobei, wenn ein SNP den $r^2$- oder LD-Schwellenwert nicht überschreitet, er nur in einer Gruppe berücksichtigt und als tagSNP selbst genommen wird.

4. Kit für die genetische Präimplantationsdiagnose von Embryomutationen in *In-vitro*-Reproduktionsverfahren, **da-**

**durch gekennzeichnet, dass** es mindestens ein elektronisches Gerät mit einem oder mehreren Prozessoren und einem Speicher umfasst, wobei der Speicher Anweisungen speichert, die bei Ausführung durch den Prozessor oder die Prozessoren bewirken, dass das elektronische Gerät das Verfahren gemäß einem der Ansprüche 1 bis 3 ausführt.

5. Computerprogrammprodukt mit Anweisungen, die so konfiguriert sind, dass sie von einem oder mehreren Prozessoren ausgeführt werden, die das elektronische Gerät des Kits von Anspruch 4 dazu veranlassen, das Verfahren gemäß einem der Ansprüche 1 bis 3 durchzuführen.

## Revendications

1. Méthode pour le diagnostic génétique préimplantatoire de mutations chez des embryons dans des procédés de reproduction *in vitro* présentant la particularité de combiner une seule biopsie avec un séquençage de nouvelle génération (NGS), les techniques de détection d'aneuploïdie (DPG-A) et l'étude de maladies monogéniques chez des embryons (DPG-M) et **caractérisée en ce qu'**elle comprend les procédés consistant en :

   un premier procédé de sélection de SNP dans lequel :

   (i) on prend comme entrée les valeurs de certains *n* SNP candidats ($t_1$ ... $t_k$) de chaque individu x, dans une région chromosomique d'intérêt et spécifiquement extraits pour une population objet d'étude ; et ce procédé étant conçu pour maximiser la situation dans laquelle l'un des parents a la valeur d'un SNP dans un état hétérozygote, tandis que l'autre parent a la valeur d'un SNP dans un état homozygote, et pour obtenir un panel de z SNP optimisés pour les deux valeurs maximisées sous la forme de matrice M dont les colonnes correspondent aux individus de la population et les lignes aux valeurs de chaque SNP pour chaque individu ;
   (ii) le procédé comprend la sélection des SNP qui sont bialléliques, les individus pouvant être représentés comme des haplotypes de longueur m formés de chaînes binaires {1,0}, 1|0 et 0|1 étant les valeurs pour les SNP hétérozygotes et 0|0 et 1|1 étant les valeurs pour les SNP homozygotes ; cette sélection étant réalisée dans toute la région chromosomique d'intérêt ;
   (iii) le procédé comprend une étape consistant à analyser les n SNP candidats dans la région et exclure les SNP qui remplissent l'une quelconque des conditions suivantes : les SNP présentant plus d'un allèle variant (SNP non bialléliques) ; les SNP dont les allèles sont différents du changement d'un seul nucléotide ; les SNP qui sont homozygotes chez au moins 99 % de la population d'intérêt ; et les SNP rares, la fréquence des allèles mineurs étant inférieure à 1 % ; et
   (iv) le procédé comprend une étape consistant à maximiser la situation dans laquelle l'un des parents a la valeur d'un SNP dans un état hétérozygote, tandis que l'autre parent a la valeur du SNP dans un état homozygote, celui-ci étant informatif, grâce à la maximisation de la valeur de deux fonctions au-dessus d'une certaine valeur de seuil :

$$\text{MaxP :} \quad p-(3p2) + (4p3) - (2p4)$$

   **Taux d'hétérozygotie** : 2pq,
   p et q étant, respectivement, les fréquences alléliques des allèles de référence et variants pour chaque SNP ;

   un deuxième procédé de sélection de SNP dans lequel :

   (v) toutes les combinaisons de SNP sont évaluées pour obtenir un ensemble minimal t de tagSNP à partir de la matrice M obtenue lors du premier procédé de sélection de SNP ; et un procédé de validation in silico du panel de tagSNP obtenu dans le deuxième procédé ;
   (vi) le procédé comprend, tout d'abord, l'organisation des SNP de la matrice *M* de la région de bloc en groupes de forte corrélation sur la base du critère de r² par paires ; la valeur de r² par paires étant calculée à partir de la fréquence allélique calculée pour la matrice *M ;*
   (vii) les SNP de différents groupes présenteront une faible corrélation, deux SNP appartenant au même groupe uniquement lorsque le r2 par paires entre eux dépassera une certaine valeur de seuil définie par l'utilisateur ; et
   (viii) la sélection de tagSNP au sein de chaque groupe est réalisée sur la base du critère de limite de détection (LD), en commençant avec k = 1 SNP et en étudiant toutes les k combinaisons possibles, en organisant les SNP au sein de chaque groupe ; et

un troisième procédé de validation, dans lequel on utilise une base de données génomique où les individus sont choisis au hasard pour effectuer 300 croisements, après quoi le nombre de tagSNP qui étaient informatifs de chaque croisement est compté et la moyenne est fournie en tant que donnée informative sur la puissance informative.

2. Méthode selon la revendication 1, dans laquelle la région chromosomique d'intérêt est définie comme une quelconque position qui est située deux mégabases au-dessus et deux mégabases en dessous du gène ou de la mutation faisant l'objet de l'étude.

3. Méthode selon l'une quelconque des revendications 1 à 2, dans laquelle si un SNP ne dépasse pas les seuils de $r^2$ ou de LD, il sera considéré uniquement dans un groupe et pris comme tagSNP par lui-même.

4. Kit pour le diagnostic génétique préimplantatoire de mutations chez des embryons dans des procédés de reproduction *in vitro,* **caractérisé en ce qu'**il comprend au moins un dispositif électronique présentant un ou plusieurs processeurs et une mémoire, dans lequel la mémoire stocke des instructions qui, lorsqu'elles sont exécutées par le ou les processeurs, amènent le dispositif électronique à exécuter la méthode selon l'une quelconque des revendications 1 à 3.

5. Produit programme informatique présentant des instructions conçues pour être exécutées par un ou plusieurs processeurs qui amènent le dispositif électronique du kit selon la revendication 4 à mettre en oeuvre la méthode selon l'une quelconque des revendications 1 à 3.

```
┌─────────────────────────────┐
│ Selection of the gene of interest │
└─────────────────────────────┘
              │
              ▼
┌─────────────────────────────┐                    ┌─────────────────────────────┐
│   Search for Polymorphisms  │                    │  Combination of different   │
└─────────────────────────────┘                    │            SNPs             │
              │                                     └─────────────────────────────┘
              ▼                                                   │
┌─────────────────────────────┐                                  ▼
│   Exclusion criteria of     │                    ┌─────────────────────────────┐
│      polymorphisms:         │                    │   Correlation Analysis r²   │
│    - Non-biallelic          │                    └─────────────────────────────┘
│    - No point mutations     │                                  │
│    - Very high frequencies  │                                  ▼
│    - Very low frequencies   │                    ┌─────────────────────────────┐
└─────────────────────────────┘                    │ Linkage disequilibrium analysis │
              │                                     └─────────────────────────────┘
              ▼                                                   │
┌─────────────────────────────┐                                  ▼
│  Selection of Polymorphisms │                    ┌─────────────────────────────┐
│      that are more likely   │                    │     Selection of tagSNP     │
│      to be  informative     │                    └─────────────────────────────┘
│  (Hardy-Weinberg equation)  │
└─────────────────────────────┘
              │
              ▼
┌─────────────────────────────┐          ┌─────────────────────────────┐
│    Matrix of candidate      │ ⟵══════⟶ │
│      polymorphisms          │          └─────────────────────────────┘
└─────────────────────────────┘
```

Final selection of 200 SNPs

Data Download
Genetics of these polymorphisms
in public databases

Simulation of crosses

Analysis of the polymorphisms
that are informative

# FIG.1

FIG.2

| SNP | Without Phasing | | | Phasing | | | | | | Type of SNP |
|---|---|---|---|---|---|---|---|---|---|---|
| | P | M | H | P | | M | | H | | |
| | | | | P1 | P2 | M1 | M2 | P1 | M1 | |
| 1 | 0/0 | 1/1 | 0/1 | 0 | 0 | 1 | 1 | 0 | 1 | Non-informative |
| 2 | 0/1 | 0/0 | 1/0 | 1 | 0 | 0 | 0 | 1 | 0 | Informative |
| 3 | 0/1 | 0/0 | 0/0 | 0 | 1 | 0 | 0 | 0 | 0 | Informative |
| 4 | 1/1 | 0/1 | 0/1 | 1 | 1 | 0 | 1 | 1 | 0 | Informative |
| 5 | 1/1 | 0/1 | 1/1 | 1 | 1 | 1 | 0 | 1 | 1 | Informative |
| 6 | 0/1 | 0/1 | 0/0 | 0 | 1 | 0 | 1 | 0 | 0 | Semi-informative |
| 7 | 0/1 | 0/1 | 0/1 | 0 | 1 | 0 | 1 | - | - | Non-informative |
| 9 | 0/0 | 0/0 | 0/0 | 0 | 0 | 0 | 0 | 0 | 0 | Non-informative |
| 10 | 1/1 | 1/1 | 1/1 | 1 | 1 | 1 | 1 | 1 | 1 | Non-informative |

# FIG.3

| Position | Father Carrier | | Mother Carrier | | Foetus Affected | | Emb. 1 Affected | | Emb. 2 Healthy | | Emb. 3 Carrier | | Emb. 4 Carrier | |
|---|---|---|---|---|---|---|---|---|---|---|---|---|---|---|
| | P1 | P2 | M1 | M2 | P1 | M1 | P1 | M1 | P2 | M2 | P1 | M2 | P2 | M1 |
| chr12:87254962 | A | G | A | A | A | A | A | A | G | A | A | A | G | A |
| chr12:87260006 | C | G | C | C | C | C | C | C | G | C | C | C | G | C |
| chr12:87302691 | A | C | A | A | A | A | A | A | C | A | A | A | C | A |
| chr12:87302747 | A | C | A | A | A | A | A | A | C | A | A | A | C | A |
| chr12:87391646 | T | A | T | T | T | T | T | T | A | T | T | T | A | T |
| chr12:87640519 | T | T | T | C | T | T | T | T | T | C | T | C | T | T |
| chr12:87674929 | C | C | C | T | C | C | C | C | C | T | C | T | C | C |
| chr12:87714749 | A | G | A | A | A | A | A | A | G | A | A | A | G | A |
| chr12:87714817 | G | G | G | C | G | G | G | G | G | C | G | C | G | G |
| chr12:87901028 | A | G | A | A | A | A | A | A | G | A | A | A | G | A |
| chr12:88238333 | C | T | C | T | C | C | C | C | T | T | C | T | T | C |
| chr12:88346589 | T | C | T | T | T | T | T | T | C | T | T | T | C | T |
| chr12:88450462 | T | T | T | A | T | T | T | T | T | A | T | A | T | T |
| chr12:88471567 | | T | | T | | | | | T | T | | T | T | |
| chr12:89400654 | T | T | T | C | T | T | T | T | T | C | T | C | T | T |
| chr12:89447754 | T | A | T | T | T | T | T | T | A | T | T | T | A | T |
| chr12:89449487 | A | G | A | A | A | A | A | A | G | A | A | A | G | A |
| chr12:89456904 | A | G | A | A | A | A | A | A | G | A | A | A | G | A |
| chr12:89459695 | G | T | G | G | G | G | G | G | T | G | G | G | T | G |
| chr12:89460093 | A | C | A | A | A | A | A | A | C | A | A | A | C | A |
| chr12:89462738 | C | T | T | T | T | T | T | T | T | T | C | T | T | T |
| chr12:89463041 | C | A | C | C | C | C | C | C | A | C | C | C | A | C |
| chr12:89468283 | G | A | G | G | G | G | G | G | A | G | G | G | A | G |
| chr12:89469954 | C | T | C | C | C | C | C | C | T | C | C | C | T | C |
| chr12:89470751 | C | G | C | C | C | C | C | C | G | C | C | C | G | C |
| chr12:89471403 | G | A | G | G | G | G | G | G | A | G | G | G | A | G |
| chr12:89473626 | A | C | A | A | A | A | A | A | C | A | A | A | C | A |
| chr12:89474352 | C | T | C | C | C | C | C | C | T | C | C | C | T | C |
| chr12:89475779 | G | A | G | G | G | G | G | G | A | G | G | G | A | G |
| chr12:89490962 | C | T | C | C | C | C | C | C | T | C | C | C | T | C |
| chr12:89491074 | G | A | G | G | G | G | G | G | A | G | G | G | A | G |
| chr12:89492878 | C | T | C | C | C | C | C | C | T | C | C | C | T | C |
| chr12:89496725 | C | C | C | T | C | C | C | C | C | T | C | T | C | C |
| chr12:89510796 | A | A | A | G | A | A | A | A | A | G | A | G | A | A |
| chr12:89510867 | T | T | T | G | T | T | T | T | T | G | T | G | T | T |
| chr12:89526316 | A | A | A | G | A | A | A | A | A | G | A | G | A | A |
| chr12:89528992 | G | G | G | A | G | G | G | G | G | A | G | A | G | G |
| chr12:89555278 | T | T | T | C | T | T | T | T | T | C | T | C | T | T |
| chr12:89562599 | A | A | A | G | A | A | A | A | A | G | A | G | A | A |
| chr12:89566879 | A | A | A | G | A | A | A | A | A | G | A | G | A | A |
| chr12:89568689 | A | A | A | G | A | A | A | A | A | G | A | G | A | A |
| chr12:89577715 | T | T | T | G | T | T | T | T | T | G | T | G | T | T |
| chr12:89585187 | T | T | T | A | T | T | T | T | T | A | T | A | T | T |
| chr12:89588699 | G | G | G | A | G | G | G | G | G | A | G | A | G | G |
| chr12:89620540 | G | G | G | T | G | G | G | G | G | T | G | T | G | G |
| chr12:89632748 | C | C | C | T | C | C | C | C | C | T | C | T | C | C |
| chr12:89669785 | A | A | A | G | A | A | A | A | A | G | A | G | A | A |
| chr12:89675376 | A | A | A | G | A | A | A | A | A | G | A | G | A | A |
| chr12:89680524 | T | C | T | C | T | T | T | T | C | C | T | C | C | T |
| chr12:89687490 | T | C | T | C | T | T | T | T | C | C | T | C | C | T |
| chr12:89690312 | G | A | G | A | G | G | G | G | A | A | G | A | A | G |
| chr12:89692857 | G | A | G | A | G | G | G | G | A | A | G | A | A | G |
| chr12:89693983 | G | T | G | T | G | G | G | G | T | T | G | T | T | G |
| chr12:89696297 | A | A | A | T | A | A | A | A | A | T | A | T | A | A |
| chr12:89697513 | T | C | T | C | T | T | T | T | C | C | T | C | C | T |
| chr12:89698922 | A | G | A | G | A | A | A | A | G | G | A | G | G | A |
| chr12:89706102 | C | C | C | A | C | C | C | C | C | A | C | A | C | C |
| chr12:89708616 | T | C | C | C | C | C | C | C | C | C | T | C | C | C |
| chr12:89708714 | A | C | A | A | A | A | A | A | C | A | A | A | C | A |
| chr12:89709057 | C | T | C | C | C | C | C | C | T | C | C | C | T | C |
| chr12:89712445 | C | T | C | C | C | C | C | C | T | C | C | C | T | C |
| chr12:89712461 | T | G | T | T | T | T | T | T | G | T | T | T | G | T |
| chr12:89712504 | A | G | A | A | A | A | A | A | G | A | A | A | G | A |
| chr12:89717805 | A | T | A | T | A | A | A | A | T | T | A | T | T | A |

# FIG.4

| | Father | | Mother | | Foetus | | Emb. 2 | | Type of error |
| | Carrier | | Carrier | | Affected | | Healthy | | |
| Position | P1 | P2 | M1 | M2 | P1 | M1 | P2 | M2 | |
|---|---|---|---|---|---|---|---|---|---|
| chr12:87254962 | A | G | A | A | A | A | G | A | |
| chr12:87260006 | C | G | C | C | C | C | G | C | |
| chr12:87302691 | A | C | A | A | A | A | A | A | ADO. This position is shown as homozygous, but it is not possible since it must be C, according to the allocation of the allele. In this case, only the M2 allele of the embryo has been amplified. |
| chr12:87302747 | A | C | A | A | A | A | C | A | |
| chr12:87391646 | T | A | T | T | T | T | A | T | |
| chr12:87640519 | T | T | T | C | T | T | T | C | |
| chr12:87674929 | C | C | C | T | C | C | C | T | |
| chr12:87714749 | A | G | A | A | A | A | G | A | |
| chr12:87714817 | G | G | G | C | G | G | G | C | |
| chr12:87901028 | A | G | A | A | A | A | G | T | Sequencing error. Neither of the parents have T, therefore it is not possible for the embryo to have it. |
| chr12:88238333 | C | T | C | T | C | C | T | T | |
| chr12:88346589 | T | C | T | T | T | T | C | T | |
| chr12:88450462 | T | T | T | A | T | T | T | A | |
| chr12:88471567 | - | T | - | T | - | - | T | T | |
| chr12:89400654 | T | T | T | C | T | T | T | T | Point of recombination. From this point, all SNPs change from the M2 allele to the M1 allele |
| chr12:89447754 | T | A | T | T | T | T | A | T | |
| chr12:89449487 | A | G | A | A | A | A | G | A | |
| chr12:89456904 | A | G | A | A | A | A | G | A | |
| chr12:89459695 | G | T | G | G | G | G | T | G | |
| chr12:89460093 | A | C | A | A | A | A | C | A | |
| chr12:89462738 | C | T | T | T | T | T | T | T | |
| chr12:89463041 | C | A | C | C | C | C | A | C | |
| chr12:89468283 | G | A | G | G | G | G | A | G | |
| chr12:89469954 | C | T | C | C | C | C | - | - | No Call. No reading has been obtained in this position, therefore it is not possible to determine the allele |
| chr12:89470751 | C | G | C | C | C | C | G | C | |
| chr12:89471403 | G | A | G | G | G | G | A | G | |
| chr12:89473626 | A | C | A | A | A | A | C | A | |
| chr12:89474352 | C | T | C | C | C | C | T | C | |

# FIG.5

Normal　　　Unbalanced　　　Unbalanced　　　Balanced

**FIG.6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2360085 T3
- US 20140274741 A
- WO 2014082032 A
- US 20150038337 A
- EP 2947156 A

**Non-patent literature cited in the description**

- **YAN et al.** *PNAS,* 2015, vol. 112 (52), 15964-15969